# EUROPÄISCHE PATENTSCHRIFT

(11) **EP 0 603 658 B1**
(45) Veröffentlichungstag und Bekanntmachung des Hinweises auf die Patenterteilung: **24.02.1999**
(21) Anmeldenummer: 93119838.6
(22) Anmeldetag: 09.12.1993
(51) Int. Cl.: A61B 5/00

(54) **Verfahren zur in-vivo-Bestimmung einer optischen Eigenschaft des Kammerwassers des Auges**
Method for in-vivo determination of an optical property of the liquid of the aqueous humor of the eye
Méthode pour déterminer in-vivo d'une propriété optique du liquide de l'humeur vitrée de l'oeil

(30) Priorität: 19.12.1992 DE 4243142
(43) Veröffentlichungstag der Anmeldung: 29.06.1994
(73) Patentinhaber: BOEHRINGER MANNHEIM GMBH, 68305 Mannheim (DE)
(72) Erfinder: Backhaus, Jürgen, Dr., D-68535 Edingen-Neckarhausen (DE); Böcker, Dirk, Dr. Dr., D-69115 Heidelberg (DE); Schrader, Bernhard, Prof. Dr., D-45259 Essen (Heisingen) (DE); Schrader, Wolfgang, Dr. med., D-79195 Kirchzarten-Burg (DE); Menzebach, Hans-Ulrich, D-45127 Essen (DE); Schmidt, Elmar, Dr., D-68167 Mannheim (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 589 191
- WO-A-92/07511
- US-A- 3 963 019
- DIABETES CARE,, Bd.5, Nr.3, Mai 1982, ALEXANDRIA,USA Seiten 254 - 258 B.RABINOWITCH ET AL 'Noninvasive Glucose Monitoring of the Aqueous Humor of the Eye: Part I. Measurement of Very Small Optical Rotations'

## Beschreibung

Die Erfindung betrifft ein Verfahren zur in-vivo-Bestimmung einer optischen Eigenschaft des Kammerwassers in der Vorderkammer des Auges. Von einer Lichtquelle wird Licht entlang einem primärseitigen Strahlengang in die Vorderkammer eingestrahlt. Aus der Vorderkammer entlang einem sekundärseitigen Strahlengang heraustretendes Licht wird mittels eines Detektors detektiert, ein Meßsignal erzeugt und die optische Eigenschaft auf Basis des Meßsignals des Detektors mittels einer Signalverarbeitungseinheit bestimmt.

Die Bestimmung der optischen Eigenschaften des Kammerwassers des menschlichen Auges ist vor allem für medizinisch-diagnostische Zwecke wichtig. Bereits seit langem wird vor allem die Möglichkeit diskutiert, die Konzentration von Glucose in der Vorderkammer (anterior chamber) mit Hilfe einer von der Glucosekonzentration abhängigen optischen Eigenschaft des Kammerwassers (aqueous humour) zu bestimmen.

In dem 1976 publizierten US-Patent 3 958 560 und in dem auf der gleichen ursprünglichen Anmeldung basierenden US-Patent 4 014 321 wird eine Vorrichtung beschrieben, durch die die optische Drehung von polarisiertem Licht beim Durchtritt durch die Vorderkammer als Maß für die darin enthaltene Glucosekonzentration bestimmt wird. Die Lichtquelle und der Detektor sollen dabei in eine Kontaktlinse integriert sein, welche auf das Auge aufgesetzt wird. Das Licht wird von einer Seite der Vorderkammer her unter einem flachen Winkel durch die Hornhaut eingestrahlt, durchläuft die Vorderkammer geradlinig und tritt auf der jenseitigen Seite wiederum unter einem flachen Winkel durch die Hornhaut aus. Der Strahlengang ist also eine gerade Linie, welche in einer Schnittebene durch die Vorderkammer eine Sekante bezüglich der Krümmung der Hornhaut bildet.

Ebenfalls bereits 1976 wurde das US-Patent 3 963 019 publiziert, in dem eine Vorrichtung beschrieben ist, bei der der Primärstrahl durch die Vorderkammer auf die Iris gerichtet ist. Das von der Iris reflektierte Licht soll hinsichtlich seiner Winkellage analysiert werden, um die optische Brechung als Maß der Glucosekonzentration zu bestimmen. Bei einer anderen Ausführungsform soll die optische Drehung des polarisiert eingestrahlten, an der Iris reflektierten Lichts gemessen werden.

Auch in der nicht vorpublizierten EP-A-0 589 191, die einen Stand der Technik nach Artikel 54 (3) EPÜ bildet, wird eine Vorrichtung beschrieben, bei der die (in diesem Fall spektroskopische) Bestimmung einer optischen Eigenschaft des Kammerwassers auf der Reflexion des Lichts an der Iris basiert.

Die nichtinvasive in-vivo-Bestimmung von Glucose durch Untersuchung des Kammerwassers hat außerordentlich große potentielle Vorteile gegenüber den derzeitig gebräuchlichen Methoden zur Glucosebestimmung, wie sie insbesondere von Diabetikern verwendet werden. Sämtliche derzeit in praktischem Gebrauch befindliche Glucose-Analysesysteme arbeiten invasiv, d.h. es ist erforderlich, für jeden Analysevorgang eine Blutprobe zu gewinnen. Dies erfolgt üblicherweise durch Einstich in die Fingerbeere. Wegen des mit dem invasiven Analyseverfahren verbundenen Schmerzes bestimmen die meisten Diabetiker ihre Blutglucosewerte nur in relativ großen Abständen.

Aus medizinischer Sicht wünschenswert wäre jedoch eine wesentlich häufigere Analyse, nach Möglichkeit mehrfach am Tage, weil nur auf diese Weise durch genau dosierte und gezielte Insulingaben die Glucosekonzentration im Blut im Normbereich gehalten werden kann. Dies wiederum ist von größter Bedeutung, weil jede Entgleisung des Glucosestatus nicht nur erhebliche akute Risiken birgt, sondern schwerwiegende Sekundärschäden des Diabetes, wie beispielsweise Verlust des Augenlichtes, nach sich ziehen kann. In medizinischen Fachkreisen ist man der Überzeugung, daß solche schwerwiegenden Diabetes-Spätschäden durch eine wesentlich häufigere und engere Kontrolle der Blutglucosekonzentration weitgehend vermieden werden könnten.

Aus diesen Gründen besteht großes Interesse an einer Möglichkeit zur nichtinvasiven in-vivo-Bestimmung der Blutglucosekonzentration. Da bekannt ist, daß die Glucosekonzentration in der Vorderkammer des Auges mit der Glucosekonzentration im Blut korreliert, hat es mehrere Versuche gegeben, die in den genannten US-Patenten beschriebene Meßtechnik weiterzuentwickeln und zu verbessern, wobei die Bemühungen sich auf die Verbesserung der optischelektronischen Meßtechnik konzentrieren. In dem Artikel "Noninvasive Optical Polarimetric Glucose Sensing Using a True Phase Measurement Technique" von G. L. Coté et al., IEEE Transactions on Biomedical Engineering, 1992, 752 bis 756, wird in der Einleitung ein Überblick über diese Entwicklung mit zahlreichen Zitaten gegeben. An den vorbekannten Meßsystemen wird jedoch kritisiert, daß Variationen aufgrund von Fluktuationen der Lichtquelle oder Wechselwirkungen des optischen Strahls mit interferierenden Teilchen bei den früher angewandten Meßtechniken zu Fehlern führen können. Es wird eine besondere optischelektronische Anordnung vorgeschlagen, um solche Probleme zu eliminieren. Diese ist auch Gegenstand der internationalen Patentanmeldung WO 92/07511.

Ein anderes meßtechnisches Verfahren zum Nachweis der sehr kleinen optischen Rotation beim Durchtritt des Lichts durch die Vorderkammer wird beschrieben in: B. Rabinovitch et al. "Noninvasive glucose monitoring of the aqueous humour of the eye: Part I. Measurement of very small optical rotations", Diabetes Care, 1982, 254 bis 258.

Aufgrund dieser und ähnlicher Arbeiten stehen außerordentlich leistungsfähige Signalverarbeitungseinheiten zur Verfügung, die grundsätzlich geeignet sind, die optischen Eigenschaften des Kammerwassers des Auges mit einer für die genannten diagnostischen Zwecke ausreichenden Genauigkeit zu bestimmen. Dennoch erscheint es nahezu ausgeschlossen, daß die zu diesem Zweck bekannten Vorrichtungen zum praktischen Einsatz gelangen könnten. Erforderlich wäre eine extreme Miniaturisierung der Bauteile von Sender und Empfänger, um sie in eine Kontaktlinse zu integrieren. Selbst wenn dies gelänge, wäre das Einsetzen und Herausnehmen der Kontaktlinse wohl für die Mehrzahl der Diabetes-Patienten bei denen es sich meist um ältere Personen handelt, eine nicht zumutbare Belastung.

Der Erfindung liegt daher die Aufgabe zugrunde, ein Verfahren zur in-vivo-Bestimmung einer optischen Eigenschaft des Kammerwassers des Auges zur Verfügung zu stellen, welches sich für eine routinemäßige Untersuchung mit möglichst geringer Belastung des Patienten eignet.

Die Aufgabe wird durch ein Verfahren gemäß Anspruch 1 gelöst.

Im Gegensatz zu dem aus dem US-Patent 3 958 560 bekannten, von Coté unter anderem in der WO 92/07511 aufgegriffenen Verfahren wird zur Messung der optischen Eigenschaften des Kammerwassers (insbesondere der Absorption oder der optischen Drehung) bei der Erfindung also die Vorderkammer nicht entlang einer Sekante der Hornhautwölbung durchstrahlt, sondern es wird die spiegelnde Reflexion an einer der Vorderkammer zugewandten Grenzfläche der Augenlinse genutzt. Die verwendete Signalverarbeitungseinheit kann ähnlich wie bei den bekannten Vorrichtungen aufgebaut sein, wobei einige bevorzugte Maßnahmen noch näher erläutert werden.

Als diffus reflektierende Grenzfläche wirkt insbesondere die Oberfläche der Iris. Das diffus reflektierte Licht ist jedoch depolarisiert. Deshalb ist bei Reflexion an der Oberfläche der Iris nur die Messung der optischen Absorption möglich, während die Messung der optischen Drehung durch das Kammerwasser mit dieser Technik nicht möglich ist. Deswegen und auch wegen der störenden Streulichtanteile bei diffuser Reflexion, wird die spiegelnde Reflexion bevorzugt an einer Grenzfläche in der Umgebung der optischen Achse des Auges (über der Pupille) detektiert. Dieser Reflex wird durch die der Vorderkammer zugewandte Begrenzung der Augenlinse erzeugt. Seine Intensität hängt von dem Unterschied der Brechungsindizes der an die reflektierende Grenzfläche angrenzenden Medien ab. Da diese Brechungsindex-Unterschiede verhältnismäßig gering sind, ist das reflektierte Signal sehr schwach. Es kann jedoch trotz seiner verhältnismäßig geringen Intensität auf Basis der hier gegebenen Erläuterungen so ausgewertet werden, daß sich nutzbare Informationen über die optischen Eigenschaften des Kammerwassers erhalten lassen.

Die Reflexion an Grenzflächen der Hornhaut und der Linse ist in der Augenheilkunde bekannt. Man kann vier Spiegelbildchen ("Purkinje-Sanson-Bildchen") eines vor dem Auge befindlichen Objektes (beispielsweise einer Kerzenflamme) beobachten, von denen zwei von der Hornhaut und zwei von der Linse des Auges entworfen werden. Dabei ist das an der Hornhautvorderfläche das bei weitem lichtstärkste Bild, d.h. die stärkste Reflexion ist an der Vorderseite der Hornhaut zu beobachten. In Relation zu diesem stärksten Reflex beträgt die Intensität der im Rahmen der vorliegenden Erfindung genutzten Reflexion an der vorderen Grenzfläche der Linse etwa 1%. Das Reflexionsvermögen der vorderen Grenzfläche der Augenlinse liegt bei weniger als 0,001. In der Augenheilkunde werden die Reflexionen an den genannten Grenzflächen verwendet, um die Struktur dieser Grenzflächen selbst zu untersuchen, nicht um irgendwelche Eigenschaften einer von dem Strahlengang durchdrungenen Substanz zu bestimmen.

Vorzugsweise werden in dem erfindungsgemäßen Verfahren die optische Drehung (ORD=optische Rotationsdispersion) oder die optische Absorption im gesamten optischen Bereich (UV=ultraviolett, VIS=sichtbar, NIR=nahes Infrarot, MIR=mittleres Infrarot und FIR=fernes Infrarot) bestimmt. Auch andere optische Eigenschaften des Kammerwassers, wie beispielsweise der Circulardichroismus, magnetisch induzierte Phänomene, der Ramaneffekt und die Fluoreszenz können im Rahmen der Erfindung bestimmt werden.

Durch das erfindungsgemäße Meßprinzip lassen sich entscheidende Vorteile erreichen.
- Eine Vorrichtung zur Durchführung des erfindungsgemäßen Verfahrens kann ähnlich wie übliche augenärztliche Untersuchungsgeräte in einer definierten Position vor dem Auge angeordnet werden. Es ist weder eine Kontaktlinse noch irgendein anderes das Auge berührendes Teil erforderlich.
- Der Lichtsender, der Detektor, die optischen Elemente der Strahlengänge und die Meß- und Auswerteelektronik der Signalverarbeitungseinheit können problemlos in einem Hand- oder Tischgerät untergebracht werden. Eine in der Praxis kaum realisierbare extreme Miniaturisierung wie bei den vorbekannten Vorschlägen ist nicht erforderlich.
- Der Strahlengang durchläuft die Vorderkammer zweimal. Dadurch ist er für die Messung der optischen Drehung ausreichend lang, obwohl das Meßlicht nur die Dicke der Vorderkammer durchläuft. Für Absorptionsmessungen ist die kürzere optische Weglänge in der Vorderkammer vorteilhaft.
- Die Abhängigkeit der optischen Weglänge in der Vorderkammer von der Justierung ist wesentlich geringer, als bei vorbekannten Geräten. Während bei dem bisher üblichen Transmissionsprinzip eine Fehljustierung von einem Zehntel Millimeter wegen der flachen Kuppenform der Vorderkammer bereits zu einer die Meßgenauigkeit entscheidend störenden Veränderung der optischen Weglänge führte, läßt sich bei der Erfindung mit verhältnismäßig einfachen Justierungsmaßnahmen eine gute Reproduzierbarkeit erreichen.
- Störungen durch Reflexion an anderen Grenzflächen, insbesondere an der Hornhaut und Streuungen an Trübungen in den von dem Strahlengang durchlaufenen Teilen des Auges werden minimiert. Bei der vorbekannten Anordnung resultierten dagegen sehr starke Störungen vor allem dadurch, daß die Hornhaut zweimal sehr schräg auf einer Sekante durchsetzt wurde.
- Es ist relativ einfach möglich, die Vorrichtung so zu gestalten, daß für beide Augen nacheinander unabhängige Meßwerte gewonnen werden können. Beispielsweise kann die Optikeinheit, wie bei augenärztlichen Geräten gebräuchlich, von einer ersten Position vor dem rechten Auge in eine zweite Position vor dem linken Auge geschwenkt werden. Hierdurch läßt sich die Meßgenauigkeit erhöhen.
- Eine weitere Steigerung der Meßgenauigkeit ist möglich, in dem man mehrere unterschiedliche optische Eigenschaften des Kammerwassers, beispielsweise die NIR-Absorption und die optische Drehung unabhängig voneinander bestimmt und als Maß für die gleiche analytische Größe, beispielsweise die Glucosekonzentration verwendet.

Die Erfindung wird im folgenden anhand von in den Figuren schematisch dargestellten Ausführungsbeispielen näher erläutert; es zeigen:
- Fig. 1: Eine Prinzipdarstellung einer Vorrichtung zur Durchführung des erfindungsgemäßen Verfahrens,
- Fig. 2: eine schematische Schnittdarstellung eines Auges zur Verdeutlichung der Reflexionen verschiedener Grenzflächen,
- Fig. 3: eine Aufsicht von der Benutzerseite her auf eine Maske zum definierten Positionieren einer für die Erfindung geeigneten Vorrichtung,
- Fig. 4: eine Prinzipdarstellung einer alternativen Ausführungsform einer für die Erfindung geeigneten Vorrichtung mit einer Signalverarbeitungseinheit zur Bestimmung der optischen Drehung,
- Fig. 5: eine graphische Darstellung eines Detektorsignals bei der Ausführungsform von Figur 4.

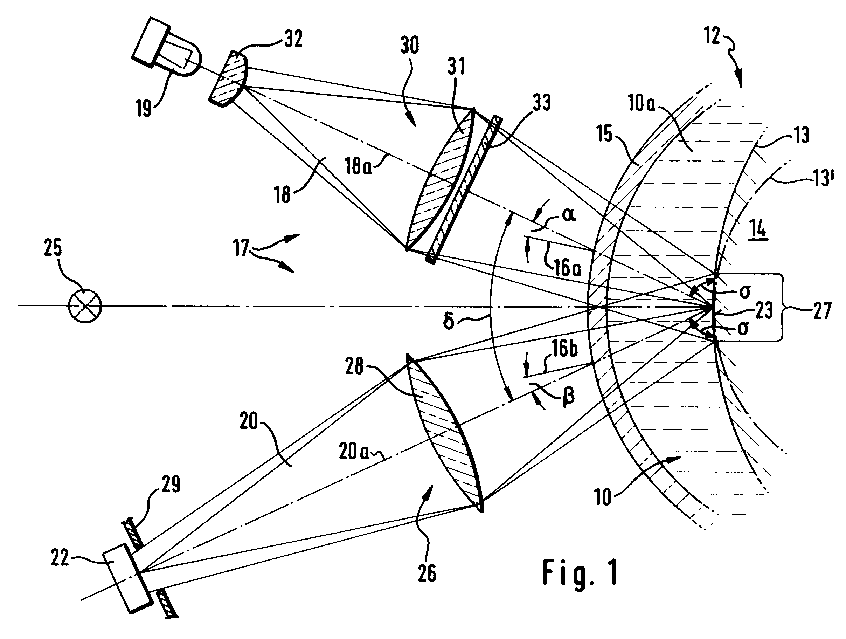

Figur 1 zeigt stark schematisiert die Vorderkammer 10 eines Auges 12. Sie ist zum Augeninneren hin von der Kapsel 13 der Augenlinse 14 und nach außen hin von der Hornhaut 15 begrenzt.

Die nicht-maßstäblich dargestellte, insgesamt mit 17 bezeichnete Optikeinheit einer Vorrichtung zur in-vivo-Bestimmung einer optischen Eigenschaft des in der Vorderkammer 10 enthaltenen Kammerwassers 10a weist einen primärseitigen Strahlengang 18 auf, um von einer Lichtquelle 19 ausgehendes Licht in die Vorderkammer 10 einzustrahlen. Das aus der Vorderkammer 10 heraustretende Licht fällt entlang einem sekundärseitigen Strahlengang 20 auf einen Detektor 22.

Der primärseitige Strahlengang 18 und der sekundärseitige Strahlengang 20 können im einzelnen sehr unterschiedlich gestaltet sein. Anhand von Figur 1 werden lediglich die für die Erfindung allgemein notwendigen oder zumindest vorteilhaften Einzelheiten erläutert.

Erfindungsgemäß wird das von einer der Vorderkammer 10 zugewandten Grenzfläche 23 der Augenlinse 14 reflektierte Licht zur Bestimmung einer optischen Eigenschaft des Kammerwassers 10a genutzt. Die Linsenkapsel 13 wird im Bereich der vorderen Grenzfläche 23 der Linse 14 von einem dünnen Häutchen gebildet. Vorder- und Rückseite dieses Häutchens geben mit der Vorderseite der Linse gemeinsam einen scharfen Reflex der auftreffenden Strahlung, der im Rahmen der Erfindung genutzt werden kann.

Die Vorrichtung ist mit einem bei augenärztlichen Geräten üblichen Fixierlicht 25 ausgestattet. Wenn der Benutzer das Fixierlicht 25 fixiert, wird er veranlaßt, maximal zu desakkomodieren, d.h. die Krümmung der vorderen Linsenkapsel 13 erreicht ihren in der Figur 1 durchgezogen dargestellten minimalen Wert. Zum Vergleich ist gestrichelt die Position der Linsenkapsel 13' im Zustand maximaler Akkomodation (Krümmungsradius 5 bis 6 mm) dargestellt. Im maximal desakkomodierten Zustand hat die vordere Linsenkapsel einen Krümmungsradius von etwa 10 mm. Der Durchmesser der Pupille beträgt 3 mm bei starker zentraler Beleuchtung von mindestens einem Auge. Bei Dunkelheit weitet sich die Pupille auf mehr als 6 mm auf.

Aufgrund des erfindungsgemäß verwendeten Reflexions-Meßprinzips durchsetzen die Zentralstrahlen 18a,20a die Hornhaut 15 nahezu im rechten Winkel, d.h. der Winkel α bzw. β zwischen der jeweiligen Flächennormalen 16a, 16b der Hornhaut 15 (d.h. der Normalen 16a, 16b, die die Hornhaut 15 in dem Punkt kreuzen, in dem die Zentralstrahlen 18a bzw. 20a die Oberfläche der Hornhaut 15 durchsetzen) und dem Zentralstrahl 18a, 20a der Strahlengänge 18,20 ist ein spitzer Winkel von vorzugsweise weniger als 45°, besonders bevorzugt weniger als 30°. Der Winkel δ zwischen den Zentralstrahlen 18a und 20a kann in weiten Bereichen variieren, wobei größere Winkel zu einem längeren Strahlengang innerhalb des Kammerwassers führen, was vielfach vorteilhaft ist, während andererseits nachteilig sein kann, daß der Winkel, unter dem die Hornhaut durchstrahlt wird, größer wird. Wesentlich ist, daß die Winkelanordnung der Lichtquelle 19 und des Detektors 22 jedenfalls so gewählt ist, daß von der Grenzfläche 23 reflektierte Lichtanteile von dem Detektor 22 detektiert werden.

Um Störungen durch Streulicht zu minimieren wird die Anordnung so gewählt, daß der Detektor 22 die von der Grenzfläche 23 ausgehende spiegelnde Reflexion erfaßt. Die Zentralstrahlen 18a,20a des primärseitigen Strahlengangs 18 und des sekundärseitigen Strahlengangs 20 treffen dabei unter dem gleichen Winkel σ auf die Grenzfläche 23.

Eine weitere Reduzierung von Störlichteinflüssen läßt sich dadurch erreichen, daß man ein optisches Abbildungssystem verwendet, um selektiv einen definierten Teilbereich 27 der Grenzfläche 23 auf den Detektor 22 abzubilden. Bei der dargestellten Ausführungsform besteht das optische Abbildungssystem 26 aus einem vereinfachend als Linse gezeichneten Objektiv 28 und einer Blende 29, die im dargestellten bevorzugten Fall im Bereich der Bildebene, also unmittelbar vor dem Detektor 22 angeordnet ist. Statt der Blende kann selbstverständlich auch eine andere definierte Begrenzung der beleuchteten Fläche des Detektors verwendet werden, etwa als Bestandteil des Detektorgehäuses.

Um einen scharfen Reflex mit möglichst hoher Intensität zu erhalten, kann es vorteilhaft sein, die Form der von dem optischen Abbildungssystem 26 erfaßten Teilfläche 27 so zu wählen, daß ihre Dimension in der durch die Zentralstrahlen 18a, 20a definierten Ebene (also in der Zeichenebene von Figur 1) kleiner ist als die hierzu senkrechte Dimension (also senkrecht zu der Zeichenebene von Fig. 1). Jedenfalls sollte die Teilfläche 27 in jeder Dimension kleiner sein als die Pupillenöffnung.

Bei der dargestellten bevorzugten Ausführungsform weist auch der primärseitige Strahlengang 18 ein optisches Abbildungssystem 30 auf. Es besteht aus einem wiederum als Linse angedeuteten Objektiv 31 und einem Kondensor 32 für das von der Lichtquelle 19 ausgehende Licht. Das Objektiv 31 bildet den Kondensor 32 auf die reflektierende Grenzfläche 23 ab, so daß die definierte Teilfläche 27 der Grenzfläche 23 gezielt beleuchtet wird. Ein Filter 33 dient dazu, die gewünschte Wellenlänge des primärseitigen Lichts zu selektieren.

Allgemein ist es zur Verbesserung des Signal-Rauschverhältnisses vorteilhaft, wenn nicht nur der sekundärseitige Strahlengang 20 so ausgebildet ist, daß eine definierte Teilfläche 27 der spiegelnden Grenzfläche 23 erfaßt wird, sondern wenn auch der primärseitige Strahlengang 18 so ausgebildet ist, daß möglichst genau die gleiche Teilfläche 27 gezielt beleuchtet wird. Die beleuchtete Fläche kann dabei zweckmäßigerweise geringfügig größer als die Teilfläche 27 sein, ist jedoch jedenfalls kleiner als die Pupillenöffnung. Statt des dargestellten optischen Abbildungssystems kann dies auch in anderer geeigneter Weise gewährleistet werden. Insbesondere steht bei Verwendung eines Lasers als Lichtquelle auch ohne fokussierendes Abbildungssystem ein scharf begrenzter Lichtstrahl zur Verfügung. Hier kann gegebenenfalls eine Strahlaufweitung vorteilhaft sein, um eine ausreichend große Teilfläche 27 zu beleuchten.

Eine weitere Verbesserung des Signal-/Rausch-Verhältnisses kann dadurch erreicht werden, daß man den primärseitigen Lichtstrahl unter dem den jeweiligen Bedingungen entsprechenden Brewster-Winkel auf die reflektierende Oberfläche richtet. Dadurch wird das reflektierte Licht in einer Richtung senkrecht zur Einfallsebene polarisiert. Dies erhöht die Reflektivität und ermöglicht eine Selektion des reflektierten Lichtes, indem man einen Polarisator mit der gleichen Ausrichtung in dem sekundärseitigen Lichtweg anordnet.

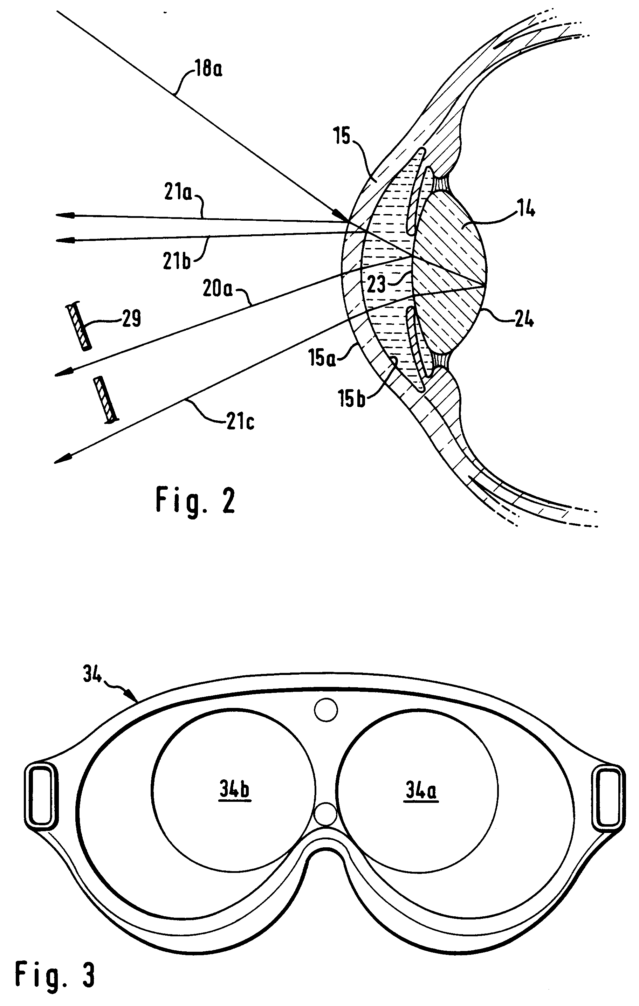

Figur 2 verdeutlicht die Reflexion eines primärseitigen Zentralstrahls 18a an vier Grenzflächen des Auges, nämlich der vorderen Grenzflächen 15a und der hinteren Grenzfläche 15b der Hornhaut 15 sowie der vorderen Grenzfläche 23 und der hinteren Grenzfläche 24 der Linse 14. Die Reflexion an diesen Grenzflächen ist die Ursache der oben erwähnten Purkinje-Sanson-Bildchen.

Durch die unterschiedlichen Auftreffwinkel auf die Grenzflächen wird der primärseitige Strahl (mit dem Zentralstrahl 18a) in unterschiedliche Raumrichtungen reflektiert. Man erkennt, daß es mit Hilfe einer lediglich schematisch angedeuteten Blende 29 möglich ist, gezielt den im Rahmen der vorliegenden Erfindung interessierenden, an der vorderen Grenzfläche 23 der Linse 14 reflektierten sekundärseitigen Strahl (mit dem Zentralstrahl 20a) auszublenden.

Figur 3 zeigt eine Maske 34 zur Positionierung der Vorrichtung am Gesicht des Benutzers. Sie kann ähnlich wie eine Tauchermaske oder Skibrille ausgebildet sein, wird jedoch auf den jeweiligen Patienten individuell - beispielsweise durch Anschäumen mit einem Hartschaum - angepaßt. In den beiden kreisrunden Fenstern 34a und 34b befinden sich lichtundurchlässige Blenden, die bei der Anpassung der Vorrichtung jeweils mit auf den Patienten individuell angepaßten Blendenlöchern für die Meßanordnung mit dem primärseitigen Strahlengang, dem sekundärseitigen Strahlengang und dem Fixierungslicht versehen werden. Die Messung der optischen Eigenschaft erfolgt zweckmäßigerweise nacheinander für beide Augen, wobei die in Figur 3 nicht dargestellte Optikeinheit 17 von dem einen Auge zu dem anderen Auge schwenkbar, verschiebbar oder umsteckbar ist. Dabei sollten nicht genutzte Blendenlöcher abgedeckt sein.

Die in Figur 1 dargestellte Ausführungsform eignet sich in Verbindung mit einer in der Figur nicht dargestellten dem Fachmann gebräuchlichen Signalverarbeitungseinheit und ergänzenden üblichen Maßnahmen bezüglich der Optikeinheit zur Bestimmung beispielsweise der optischen Absorption des Kammerwassers 10a. Die Absorptionsmessung basiert üblicherweise auf einem Vergleich des in die Vorderkammer 10 eingestrahlten und des aus ihr heraustretenden Lichts. Zu diesem Zweck weist zweckmäßigerweise der primärseitige Strahlengang 18 eine in Figur 1 nicht dargestellte Strahlaufteilung auf, durch die ein Referenzstrahl gewonnen wird. Dies kann beispielsweise mit Hilfe eines stationären Strahlteilers oder mit Hilfe eines temporär in den primärseitigen Strahlengang 18 hineinragenden Spiegels geschehen. Eine nähere Erläuterung der Absorptionsmessung ist nicht erforderlich, weil geeignete Meßverfahren dem Fachmann bekannt sind und ohne besondere Probleme auf den vorliegenden Fall übertragen werden können.

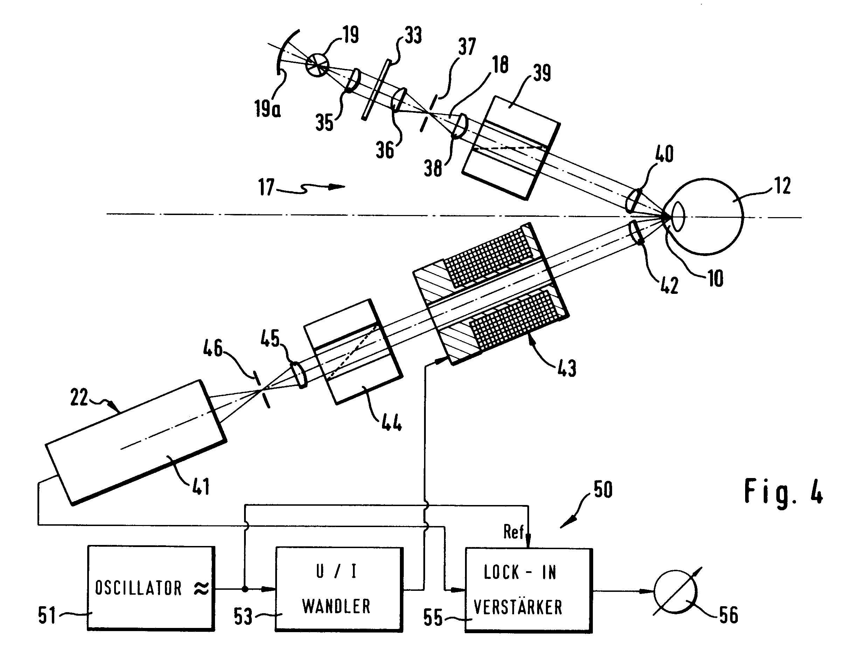

Figur 4 zeigt eine Ausführungsform, mit der die von der Glucosekonzentration in dem Kammerwasser 10a abhängige optische Drehung bestimmt werden kann. Auch diese Ausführungsform ist nur ein Beispiel. Es können andere Signalverarbeitungseinheiten zur Bestimmung der optischen Drehung verwendet werden, wie sie beispielsweise aus der einleitend erwähnten Literatur bekannt sind.

Der primärseitige Strahlengang 18 der Optikeinheit 17, der von einer Lichtquelle 19 mit Konkavspiegel 19a ausgeht, führt durch eine erste Plankonvexlinse 35, einen Filter 33, eine zweite Plankonvexlinse 36, eine Irisblende 37, eine dritte Plankonvexlinse 38, einen Polarisator 39 und eine vierte Plankonvexlinse 40 in die Vorderkammer 10 eines Auges 12. Der Detektor 22 ist im dargestellten Fall als Photomultiplier 41 ausgebildet. Der von der Vorderkammer 10 zu dem Photomultiplier 41 führende sekundärseitige Strahlengang führt durch eine fünfte Plankonvexlinse 42, einen Faraday-Modulator 43, einen als Analysator dienenden zweiten Polarisator 44, eine sechste Plankonvexlinse 45 und eine zweite Irisblende 46. Der Faraday-Modulator 43 und der Photomultiplier 41 sind an die insgesamt mit 50 bezeichnete Signalverarbeitungseinheit angeschlossen.

Die in Figur 4 dargestellte Vorrichtung funktioniert folgendermaßen.

Das von der Lichtquelle 19 erzeugte Licht sollte sein Maximum nach Möglichkeit im gewünschten Wellenlängenbereich haben. Bei der erfindungsgemäßen Reflexionsmessung wird im Gegensatz zu vorbekannten Verfahren vorzugsweise mit relativ kurzen Wellenlängen im blauen Bereich des sichtbaren Lichtes oder im UV-Bereich gearbeitet, da die optische Drehung zu kurzen Wellenlängen hin zunimmt. Zwar ist auch die Störstrahlung für Licht im blauen Bereich stärker als im roten. Diese Störung läßt sich jedoch bei der erfindungsgemäßen Strahlengang-Konzeption besser unterdrücken als bei vorbekannten Anordnungen.

Als Lichtquelle geeignet ist insbesondere ein Laser bzw. eine Laserdiode, soweit diese im gewünschten Wellenlängenbereich zu vertretbaren Kosten zur Verfügung steht. Es ist jedoch auch eine andere Lichtquelle, beispielsweise - wie dargestellt - ein Jodquarzlampe geeignet. Das von der Lichtquelle 19 ausgehende Licht wird durch die Linsen 35,36 auf die Blende 37 abgebildet und zugleich durch den Filter 33 gefiltert, wobei die Durchlaß-Wellenlänge des Filters vorzugsweise zwischen 290 nm und 440 nm liegt. Die Linsen 38 und 40 bilden zusammen mit den Linsen 35 und 36 ein optisches Abbildungssystem, durch das die Lichtquelle 19 auf die reflektierende Grenzfläche abgebildet wird. Zwischen den Linsen 38 und 40 ist der Strahlengang weitgehend parallel. Hier findet eine lineare Polarisation durch den Polarisator 39 statt. Da die Transmission der üblichen Polarisationsfolien im UV zu gering ist, werden für den Polarisator 39 ebenso wie für den Analysator 44 Polarisationsprismen verwendet.

Auch in dem sekundärseitigen Strahlengang 20 befindet sich zwischen den Linsen 42 und 45 ein Abschnitt mit im wesentlichen parallelem Strahlengang, in welchem außer dem manuell oder elektrisch drehbaren Analysator 44, der Faraday-Modulator 43 angeordnet ist. Die Funktion eines Faraday-Modulators basiert auf einem optischen Dreheffekt, den viele Materialien aufweisen, wenn sie von einem Magnetfeld durchsetzt werden, wobei die Strahlrichtung parallel zu den magnetischen Feldlinien verläuft. Der Betrag dieses Effekts wird von einer Materialkonstante, der sogenannten Verdet-Konstante, bestimmt. Bringt man ein solches Material in eine von einem elektrischen Strom durchflossene Spule, so läßt sich die Stärke und Richtung der optischen Drehung durch Variation des Stroms einstellen.

Im vorliegenden Fall dient der Faraday-Modulator 43 dazu, die Polarisationsrichtung des Lichtstrahls zu modulieren. Zu diesem Zweck weist die Auswerteeinheit 50 einen Oszillator 51 auf, der ein Sinussignal erzeugt, welches an einem Spannungs-Stromwandler 53 anliegt. Dieser speist den Faraday-Modulator mit einer seinem Eingangssignal proportionalen Stromstärke.

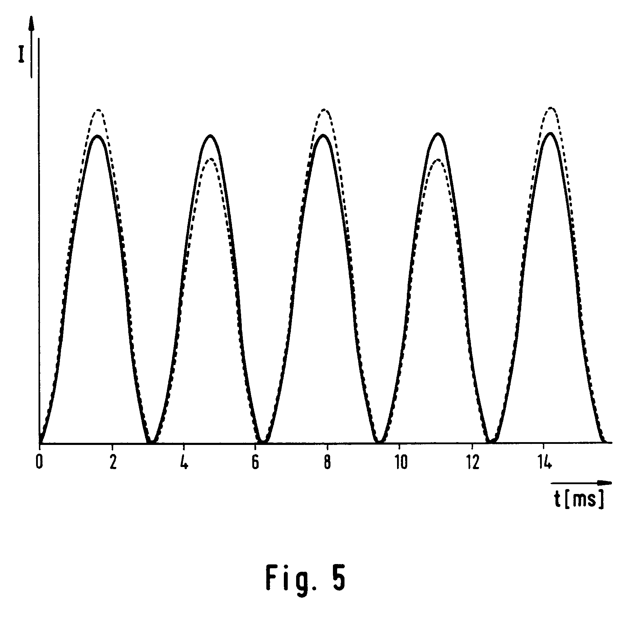

Figur 5 zeigt den von dem Detektor 22 gemessenen Verlauf des Signals I für den Fall, daß der Analysator senkrecht zur Polarisationsebene des Polarisators orientiert ist (Dunkelstellung). Die periodische Modulation durch den Faraday-Modulator 43 mit einer Frequenz f führt dazu, daß hinter dem Analysator 44 Helligkeitsmaxima mit einer Frequenz 2f auftreten. Die durchgezogene Linie von Figur 5 zeigt den Signalverlauf ohne Berücksichtigung der optischen Drehung durch die Glucose.

Damit ist die Modulationsfrequenz f in dem von dem Detektor 22 erfaßten Signal enthalten. Mit Hilfe eines Lock-In-Verstärkers kann dieser Anteil gezielt erfaßt und verstärkt werden. Zu diesem Zweck liegt die Frequenz des Oszillators 51 am Referenzeingang des Lock-In-Verstärkers 55.
Die optische Drehung durch Glucose in der Vorderkammer 10 führt dazu, daß die durch die Modulation verursachten aufeinanderfolgenden Helligkeitsmaxima nicht mehr gleich groß sind (Fig. 5, gepunktete Linie). Der Lock-In-Verstärker erzeugt ein mit der Differenz der Helligkeitsmaxima und somit mit der optischen Drehung durch die Glucose korrelierendes Ausgangssignal, welches auf einem Display 56 angezeigt oder in üblicher Weise weiterverarbeitet werden kann. Der Nullpunkt läßt sich dabei mit Hilfe einer an der Stelle der Augenlinse positionierten Fläche, welche ein ähnliches Reflexionsvermögen wie die Grenzfläche 23 hat, aber keine optische Drehung verursacht, einregeln.

Die vorstehend anhand der Figuren 2 und 3 beschriebene Ausführungsform einer für die Erfindung geeigneten elektronischen Auswerteeinheit unter Verwendung eines Faraday-Modulators ist insofern besonders bevorzugt, als keine beweglichen Teile erforderlich sind und nur ein Faraday-Modulator benötigt wird. Sie eignet sich deswegen besonders für relativ einfach aufgebaute zuverlässige Geräte, wie sie ein Diabetiker zur routinemäßigen Untersuchung seiner Glucosewerte benötigt. Grundsätzlich geeignet sind jedoch auch andere meßtechnische Verfahrensweisen, mit denen sehr kleine optische Drehungen erfaßt werden können, wie sie beispielsweise in den einleitend genannten Literaturstellen näher erläutert werden.

## Patentansprüche

1. Verfahren zur in-vivo-Bestimmung einer optischen Eigenschaft des Kammerwassers in der Vorderkammer des Auges, bei welchem
Licht von einer Lichtquelle (19), entlang einem primärseitigen Strahlengang (18) in die Vorderkammer (10) eingestrahlt wird,
Licht, welches entlang einem sekundärseitigen Strahlengang (20) aus der Vorderkammer (10) heraustritt, mittels eines Detektors detektiert und ein Meßsignal erzeugt wird und
die optische Eigenschaft auf Basis des Meßsignals des Detektors mittels einer Signalverarbeitungseinheit bestimmt wird,
**dadurch gekennzeichnet, daß**
der primärseitige Strahlengang (18) auf die der Vorderkammer (10) zugewandte Grenzfläche (23) der Augenlinse (14) gerichtet wird,
die Winkelanordnung der Lichtquelle (19) und des Detektors (22) so gewählt wird, daß von der vorderen Grenzfläche (23) der Augenlinse (14) spiegelnd reflektierte Lichtanteile detektiert werden, und
die optische Eigenschaft des Kammerwassers mittels der Signalverarbeitungseinheit aus dem Meßsignal des spiegelnd reflektierten Lichts bestimmt wird.

2. Verfahren nach Anspruch 1, **dadurch gekennzeichnet,** daß die spiegelnd reflektierende Grenzfläche in der Nachbarschaft der optischen Achse vor der Pupille liegt.

3. Verfahren nach Anspruch 1 oder 2, **dadurch gekennzeichnet,** daß die spiegelnd reflektierende Grenzfläche (23) mittels eines in dem sekundärseitigen Strahlengang angeordneten optischen Abbildungssystems (26) auf den Detektor (22) abgebildet wird.

4. Verfahren nach Anspruch 3, **dadurch gekennzeichnet,** daß die Abbildung des spiegelnd reflektierten Lichtes mittels einer einen Bestandteil des optischen Abbildungssystems (26) bildenden definierten Begrenzung (29) der beleuchteten Fläche des Detektors (22) auf eine definierte Teilfläche (27) der Grenzfläche (23) begrenzt wird.

5. Verfahren nach Anspruch 4, **dadurch gekennzeichnet,** daß die von dem optischen Abbildungssystem (26) des sekundärseitigen Strahlengangs (20) erfaßte Teilfläche (27) gezielt beleuchtet wird.

6. Verfahren nach einem der vorhergehenden Ansprüche, **dadurch gekennzeichnet,** daß die Lichtquelle (19) und der Detektor (22) Bestandteile einer Vorrichtung sind, die eine individuell an das Gesicht eines bestimmten Benutzers angepaßte Maske (34) aufweist und die Maske zur Bestimmung der optischen Eigenschaft gegen das Gesicht gedrückt wird, um die Lichtquelle (19) und den Detektor (22) definiert in Bezug auf die Vorderkammer (10) zu positionieren.

7. Verfahren nach einem der vorhergehenden Ansprüche, **dadurch gekennzeichnet,** daß die optische Eigenschaft die optische Drehung ist.

8. Verfahren nach Anspruch 7, **dadurch gekennzeichnet,** daß das Licht mit einer Wellenlänge zwischen 290 nm und 440 nm in die Vorderkammer (10) eingestrahlt wird.

9. Verfahren nach einem der Ansprüche 1 bis 6, **dadurch gekennzeichnet,** daß die optische Eigenschaft die optische Absorption ist.

10. Verfahren nach den Ansprüchen 7 und 9, **dadurch gekennzeichnet,** daß sowohl die optische Drehung als auch die optische Absorption bestimmt wird.

## Claims

1. Method for the in vivo determination of an optical property of the aqueous humour in the anterior chamber of the eye, comprising
irradiating light from a light source (19) along a primary-side light path (18) into the anterior chamber (10),
detecting light emerging from the anterior chamber (10) along a secondary-side light path (20) by means of a detector (22) and generating a measurement signal, and
determining the optical property on the basis of the measurement signal of the detector by means of a signal processing unit,
**characterised in that**
the primary-side light path (18) is directed onto the interface (23) of the eye lens (14) facing the anterior chamber (10),
the angular arrangement of the light source (19) and the detector (22) is such that specular reflection of light from the front interface (23) of the eye lens (14) is detected, and
the optical property of the aqueous humour is determined by means of the signal processing unit from the measurement signal of the specular reflected light.

2. Method according to claim 1, characterised in that the specular reflecting interface is located in the vicinity of the optical axis in front of the pupil.

3. Method according to claim 1 or 2, **characterised in that** the specular reflecting interface (23) is imaged onto the detector (22) by means of an optical imaging system (26) provided in the secondary-side light path (20).

4. Method according to claim 3, **characterised in that** the imaging of the specular reflected light is limited to a defined surface segment (27) of the interface (23) by means of a defined restriction (29) of the illuminated surface of the detector (22), said restriction being an element of the optical imaging system (26).

5. Method according to claim 4, **characterised in that** the defined surface segment (27) to which the optical imaging system (26) of the secondary-side light path (20) is limited is selectively illuminated.

6. Method according to any one of the preceding claims, **characterised in that** the light source (19) and the detector (22) are components of a device which comprises a mask (34) adapted individually to the face of a particular user, and the mask is pressed against the face for the determination of the optical property, in order to locate the light source (19) and the detector (22) in a defined position with respect to the anterior chamber (10).

7. Method according to any one of the preceding claims, **characterised in that** the optical property is the optical rotation.

8. Method according to claim 7, **characterised in that** the light is irradiated into the anterior chamber (10) with a wavelength between 290 nm and 400 nm.

9. Method according to any one of claims 1 to 6, **characterised in that** the optical property is the optical absorption.

10. Method according to claims 7 and 9, **characterised in that** both the optical rotation and the optical absorption are determined.

## Revendications

1. Procédé pour la détermination in-vivo d'une caractéristique optique de l'humeur aqueuse dans la chambre antérieure de l'oeil, dans lequel
de la lumière venant d'une source de lumière (19) est irradiée dans la chambre antérieure (10) le long d'une trajectoire de rayon du côté primaire (18),
de la lumière, qui sort de la chambre antérieure (10) le long d'une trajectoire du côté secondaire de rayon (20), est détectée au moyen d'un détecteur et un signal de mesure est produit, et
la caractéristique optique est déterminée sur base du signal de mesure du détecteur au moyen d'une unité de traitement de signal,
caractérisé en ce que
la trajectoire de rayon du côté primaire (18) est dirigée vers la surface limite (23) du cristallin (14) tournée vers la chambre antérieure (10),
la disposition angulaire de la source de lumière (19) et du détecteur (22) est choisie de façon à ce que des parts de lumière réfléchies par miroitement de la surface limite antérieure (23) du cristallin (14) sont détectées, et
la caractéristique optique de l'humeur aqueuse est déterminée au moyen de l'unité de traitement de signal au départ du signal de mesure de la lumière réfléchie par miroitement.

2. Procédé selon la revendication 1, caractérisé en ce que la surface limite réfléchissante par miroitement se trouve à proximité de l'axe optique de la pupille.

3. Procédé selon la revendication 1 ou 2, caractérisé en ce que la surface limite réfléchissante par miroitement (23) est représentée sur le détecteur (22) au moyen d'un système de représentation optique (26) disposé dans la trajectoire de rayon du côté secondaire.

4. Procédé selon la revendication 3, caractérisé en ce que la représentation de la lumière réfléchie par miroitement est limitée à une surface partielle (27) définie de la surface limite (23) au moyen d'une limite définie (29) de la surface éclairée du détecteur (22) formant une partie intégrante du système de représentation optique (26).

5. Procédé selon la revendication 4, caractérisé en ce que la surface partielle (27) saisie par le système de représentation optique (26) de la trajectoire de rayon du côté secondaire (20) est éclairée de manière ciblée.

6. Procédé selon l'une quelconque des revendications précédentes, caractérisé en ce que la source de lumière (19) et le détecteur (22) font partie intégrante d'un dispositif qui présente un masque (34) adapté individuellement au visage d'un utilisateur déterminé et en ce que le masque est appuyé contre le visage pour détermination de la caractéristique optique, afin de positionner la source de lumière (19) et le détecteur (22) de manière définie par rapport à la chambre antérieure (10).

7. Procédé selon l'une des revendications précédentes, caractérisé en ce que la caractéristique optique est la rotation optique.

8. Procédé selon la revendication 7, caractérisé en ce que la lumière est irradiée dans la chambre antérieure (10) avec une longueur d'ondes comprise entre 290 nm et 440 nm.

9. Procédé selon l'une quelconque des revendication 1 à 6, caractérisé en ce que la caractéristique optique est l'absorption optique.

10. Procédé selon les revendications 7 et 9, caractérisé en ce qu'on détermine tant la rotation optique que l'absorption optique.
